# EUROPEAN PATENT SPECIFICATION

(11) **EP 1 847 621 B1**
(45) Date of publication and mention of the grant of the patent: **07.06.2017**
(21) Application number: 06425275.2
(22) Date of filing: 21.04.2006
(51) Int. Cl.: C12P 19/02, C13K 1/02, C12P 7/10

(54) **Process and plant for the production of fermentable sugars from cellulose material**
Verfahren und Vorrichtung zur Herstellung von fermentierbaren Zuckern aus cellulosehaltigem Material
Procédé et installation destinés à la production de sucre fermentable à partir de matières contenant de la cellulose

(43) Date of publication of application: 24.10.2007
(73) Proprietor: AMBIENTE E NUTRIZIONE S.r.l., 20089 Rozzano (MI) (IT)
(72) Inventor: CEREA, Giuseppina, 20089 Rozzano (Milano) (IT)
(74) Representative: Ferreccio, Rinaldo

(56) References cited:
- EP-A- 0 884 391
- EP-A1- 0 710 670
- EP-A1- 0 711 505
- EP-A2- 0 780 510
- WO-A2-2005/024037
- US-A- 4 409 329
- US-A- 4 880 473

## Description

### Field of application

In its most general aspect, the present invention refers to the field of the treatment of cellulose material and in particular the invention regards a process and a plant for the production of fermentable sugars, here identified as simple sugars, and/or bioethanol from such material.

By cellulose material it is here intended that material comprising the lignocellulose portion of the biomass that is the fibrous portion of vegetable origin of the biomass and comprising wood, cut branches and rejects from the wood industry in general, forage, vegetables generally including their fruits, seeds and shells comprising the cereals, agricultural and zootechnic residues, rejects from agrobusiness, municipal solid waste, but also paper material, generally as rejects of the paper industry and the like.

### Prior art

The need to have available energy sources which are alternative to traditional sources is known, as is in particular the need to have available biofuels as substitutes of the fossil fuels.

The advantageous characteristics of such biofuels are well known, such as for example bioethanol and biogas, which can be produced from widely available and economical basic material, even by recycling waste materials, and which moreover lead to reduced environmental impact both relative to the production and above all for that concerning their use, for example, as fuel employed in the transportation sector.

To satisfy the aforesaid need, the prior art makes available numerous processes which use the biomass as starting material, which as is known comprises substances of biological origin including materials and residues of agricultural and forest origin, secondary products and rejects of agrobusiness, refluent of zootechnic origin, but also urban waste, the algae and various vegetable species employed in the treatment of organic sewage. Production processes are known for example of bioethanol obtained through the alcoholic fermentation of simple sugars, such as mainly hexose and pentose monosaccharides, in turn obtained through enzymatic digestion (saccharification) of the fibrous portion present in the biomass, which is appropriately treated.

Examples of such processes are described in the patents US 5,837,506 and EP 0 884 391. WO 2005/024037 describes a traditional cellulose hydrolysis process with acid pretreatment and enzyme hydrolysis. The simple sugars are finally fermented to ethanol. The pretreatment reactor is optionally a turbulator. EP 0 780 510 describes the use of a turbo reactor for the production of cellulose from vegetable materials comprising a heat treatment of said material, centrifuged in a thin layer and maintained in a state of high turbulence, with at least one digesting agent. EP 0 710 670 describes the use of a turbo reactor for the hydrolysis of starch by acids comprising a heat treatment of said material, arranged in a thin layer and maintained in a state of high turbulence.

### Summary of the invention

The invention is best described by the independent claims 1 and 17. One object of the present invention is that of making available an alternative process to the processes made available by the prior art for the production of simple sugars and/or bioethanol starting from a cellulose material, in particular a process which achieves a high yield of conversion of said cellulose material into fermentable sugars and/or bioethanol, that is a process through which the entire lignocellulose portion present in said cellulose material is utilised for the production of such sugars and/or bioethanol.

The aforesaid lignocellulose portion essentially consists of the fibrous portion of vegetable origin of the biomass and mainly comprises lignin, cellulose and hemicellulose.

A further object of the present invention is to make available a process of the aforesaid type which is particularly simple and economically advantageous.

Such objects are achieved, according to the invention, from a process for the production of simple sugars comprising the steps of:
feeding a continuous flow of the aforesaid cellulose material or a mixture composed of said cellulose material and water to a turbo-reactor comprising a horizontal axis cylindrical tubular body,
provided with at least one opening for introducing the cellulose material or aqueous mixture of cellulose material and reagents, at least one discharge opening of the treated material, a heating jacket for bringing the inner wall of the tubular body to a predetermined temperature, a bladed rotor, rotatably supported in the cylindrical tubular body, where it is put in rotation at a speed in the range of 15-40 m/s, so as to disperse said continuous flow of cellulose material or said mixture in a flow of cellulose material particles,
feeding into said turbo-reactor in co-current with said flow of cellulose material or with said mixture, a continuous and finely subdivided flow of a reagent capable of breaking the integrity of the molecular structure of said cellulose material, making it particularly accessible for being digested during an enzymatic treatment,
centrifuging said particles of cellulose material and said reagent against the inner wall of the turbo-reactor, heated to a temperature of at least 150°C, with the formation of a highly turbulent, dynamic and thin tubular fluid layer, wherein the cellulose material particles and said reagent are mechanically maintained in close contact by the blades of said bladed rotor,
making said cellulose material and said reagent react in said thin layer while it advances in substantial contact with said inner wall of the turbo-reactor towards the discharge opening,
continuously discharging a flow of treated cellulose material,
sending said flow of treated cellulose material to an enzymatic digestion step (saccharification) with the addition of a sufficient quantity of enzymes, said enzymes being specific for hydrolysing said treated cellulose material with the obtainment of said simple sugars, mainly fermentable monomeric sugars such as hexoses and pentoses, but also disaccharides, wherein said continuous flow of cellulose material or said aqueous mixture of cellulose material and said reagent flow are premixed with each other in a turbo-mixer placed upstream of said turbo reactor, said turbo-mixer advantageously comprises a horizontal-axis cylindrical tubular body, provided with inlet openings of said cellulose material or said mixture and said reagent, at least one discharge opening of the cellulose material premixed with said reagent, a cooling/heating jacket for bringing the inner wall of the tubular body to a predetermined temperature in the range of 10-100°C, and a bladed rotor, rotatably supported in the cylindrical tubular body where it is put in rotation at a peripheral speed in the range of 15-40 m/s, so as to premix the continuous flow of said cellulose material or said aqueous mixture of cellulose material with said reagent flow in a flow of premixed material.

The premixed material flow discharged from said turbomixer is then stored in a hopper connected to at least one feeding pump for feeding said premixed cellulose material flow into said turbo-reactor at a pressure in the range of approximately 1-15 bars.

In the case of premixing in said turbomixer, the aforesaid reagent capable of breaking the integrity of the molecular of the cellulose material is preferably sulphuric acid or a solution of sulphuric acid fed in a percentage in the range of 1-5% in relation to the weight of the cellulose material.

It should be added that in the aforesaid case, the process according to the invention may also comprise a neutralisation step, for example, by means of adding lime, to block the breaking reaction of the molecular integrity of the lignocellulose portion of the cellulose material, preventing the formation of undesired reaction products such as furfurals.

The furfurals in fact inhibit the enzymatic hydrolysis of the complex sugars and also the alcoholic fermentation of the simple sugars which lead to the formation of bioethanol.

Through such neutralisation step, which may advantageously be conducted in a conventional manner before the aforesaid enzymatic digestion step, the pH of the treated cellulose material flow is brought to a value in the range of 1-6.

Advantageously, according to the invention, said reagent capable of breaking the integrity of the molecular structure of the cellulose material may also be an hydrogen peroxide solution.

In the latter case, the hydrogen peroxide solution is advantageously fed directly into said turbo-reactor in co-current with said cellulose material flow or said aqueous mixture of cellulose material.

The hydrogen peroxide solution may be introduced in the turbo-reactor right at the inlet, since in such case the bladed rotor causes an effective nebulisation and centrifugation, consequently ensuring its insertion in the dynamic, turbulent, thin layer of cellulose material particles to be treated in a dispersed condition.

In such a manner, the closest contact possible between the particles and the oxygenated water is favoured, and this permits greatly increasing the effectiveness of the treatment.

Alternatively, or in addition to the aforesaid insertion through an inlet opening, the introduction of hydrogen peroxide can also be carried out through openings for the nebulisation of hydrogen peroxide placed at different levels along the length of the cylindrical tubular body.

That said above regarding the hydrogen peroxide is valid for any employed reagent capable of breaking the integrity of the molecular structure of the cellulose material, even if in the case of sulphuric acid and similar reagents a process comprising the aforesaid premixing step is preferred, as described above.

The hydrogen peroxide solution is fed at a pressure in the range of about 1 bar to about 15 bars and is composed of hydrogen peroxide to at least 10 volumes (i.e. 3% titre), preferably to 30-40 volumes.

The weight ratio between hydrogen peroxide employed and cellulose material varies from 1:2 to 1:20.

The residence time of said flow of cellulose material or said mixture of water and cellulose material, with said reagent flow in the turbo-reactor, is a variable time depending on the type and quantity of employed reagent, as well as on the temperature of the inner wall of the turbo-reactor.

The reaction in the turbo-reactor has in fact the object of making the cellulose material particularly accessible, particularly its fibrous or lignocellulose portion, for the subsequent step of enzymatic hydrolysis, mainly through the breaking of the crystalline structure of the lignin present in the cellulose material.

For example, employing a solution of 5% hydrogen peroxide (16.6 volumes), fed into the turbo-reactor at double the flow rate of the cellulose material flow, the aforesaid residence time is preferably in the range of about 3 minutes to about 30 minutes, and more preferably is a residence time of about 10 minutes.

Advantageously, then, the turbo-reactor is a substantially sealed turbo-reactor which is capable of maintaining an inner pressure at a value of about 15 bars, or rather a pressure essentially corresponding to that of the flow or flows fed into it.

Regarding the aforesaid step of enzymatic digestion or saccharification, it should be said that it is carried out at a temperature in the range of 35-45°C, preferably 40°C, and in any case corresponding with a temperature at which the activity of the employed enzymes is sufficient to achieve the aforesaid hydrolysis reaction, through which the complex sugars (polysaccharides and/or oligosaccharides) are transformed into simple fermentable sugars.

For such purpose, the flow of treated cellulose material discharged from the turbo-reactor may be subjected to cooling, also for example by dilution.

In any case it should be said that the temperature of the treated cellulose material flow at the turbo-reactor's discharging mainly depends on the temperature of the inner wall of the turbo-reactor and on the residence time of the same cellulose material flow at its interior.

Moreover, it should be said that the aforesaid step of enzymatic digestion is carried out for a time which varies in the range of 10 minutes to 36 hours, depending on the enzymes employed, that is, on their activity and the quantity used.

In particular, any enzyme of known type can be employed which is capable of inducing the aforesaid step of enzymatic digestion or saccharification, and only as an example we mentioned the enzymes available with the trademark of Celluclast® and Liquozyme© SC.

The enzymatic digestion step can moreover be conducted through a plurality of substeps of enzymatic digestion, in each of which predetermined quantities of enzymes may be added, even of different type.

Regarding once again the cellulose material, it should be said that it is fed in a flow with a rather fine granulometry, wherein preferably no more than 10% of the cellulose material is of size greater than 1000 microns and in any case less than 1500 microns.

Still more preferably, at least 50% of the cellulose material has a granulometry comprised between 200 and 1000 microns.

It should be said that the process according to the invention may involve a dilution, generally with water, of the flow of treated cellulose material discharged from the turbo-reactor, or said flow discharged from the turbo-reactor can be if necessary dried.

This is verified when, for a flow of treated cellulose material discharged from the turbo-reactor, a mass of treated cellulose material is desired with specific fluidity and/or moisture characteristics.

On the other hand, as it appears from that stated above, such characteristics can be attained by causing an appropriate mixing of the cellulose material fed into the turbo-reactor together with water.

Regarding the temperature of the inner wall of the turbo-reactor, which as previously set forth is heated to at least 150°C, it should be said that it is preferably in the range of about 220 to about 300°C, best if about 260°C.

The process according to the invention may also comprise, subsequently or simultaneously with the aforesaid enzymatic digestion step, a step of alcoholic fermentation, of known type, with the transformation of said simple sugars into bioethanol.

In another of its aspects, the present invention refers to a plant for carrying out the above illustrated process which comprises a turbo-reactor of the type described above and optionally a turbo-mixer, it too of the above described type placed upstream of the turbo-reactor.

Such plant may moreover comprise a hopper placed downstream of the turbo-mixer and a pump connected with said hopper and placed upstream of said turbo-reactor, respectively for the collection of the aforesaid flow of premixed cellulose material discharged from the turbo-mixer and for feeding the flow collected in said hopper into the turbo-reactor at a pressure in the range of about 1 bar to about 15 bars.

It should be said that, advantageously, the present process is particularly effective, giving high conversion yields.

In particular, it should be noted that such yields are surprising high in the case a solution of hydrogen peroxide is employed as reagent inside the turbo-reactor.

The use of hydrogen peroxide in the turbo-reactor moreover has the advantage of not influencing the activity of the enzymes employed in the subsequent step of enzymatic digestion, to which the treated cellulose material discharged from the turbo-reactor is subjected.

The hydrogen peroxide in fact is completely deactivated after the reaction in the turbo-reactor.

Consequently, no neutralisation step is requested for the cellulose material before the saccharification step, as instead is in the case of the use of sulphuric acid which must be neutralised with an oxide or alkaline hydroxide or earth alkaline, for example with lime.

Again, the residual cellulose material which is obtained after the step of enzymatic digestion, in case the aforesaid reaction in the turbo-reactor was conducted with hydrogen peroxide, is composed of an active fibre which advantageously may be employed as a dietary supplement in the human diet, for example as a dietary supplement of fibres and salts.

Further advantages and features of this invention will be more evident from the description of an embodiment of a process according to the invention, hereinafter provided with reference to the attached drawing, given for illustrative and non-limiting purposes..

### Brief description of the drawings

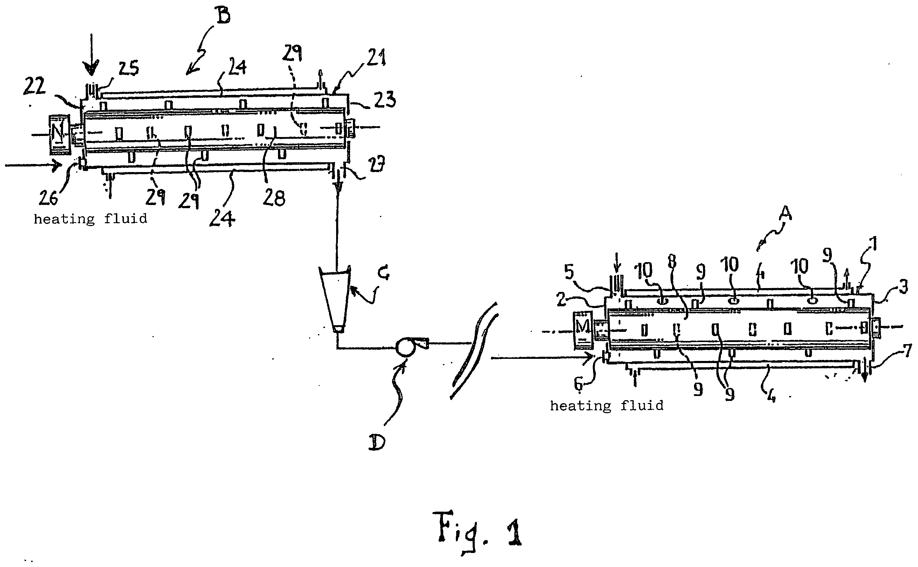

In figure 1, several details are schematically represented of a plant for the production of simple sugars and/or bioethanol from a cellulose product according to the process of the invention.

### Detailed description of the invention

With reference to figure 1, the turbo-reactor A is essentially composed of a cylindrical tubular body 1, which is substantially sealed for pressure values up to 15 bars, closed at opposite ends by bottoms 2, 3 and coaxially equipped with a heating jacket 4 intended to be run through by a heating fluid, for example steam under pressure or diathermic oil, to maintain the inner wall of the body 1 at a predetermined temperature.

The tubular body 1 is provided with inlet openings 5, 6 of the cellulose material to be treated or a mixture of it with water and respectively of the reagent employed for breaking the crystalline structure of the fibre present in the cellulose material, as well as a discharge opening 7 of the treated cellulose material.

In the tubular body 1, a bladed rotor 8 is rotatably supported whose blades 9 are helically arranged and are oriented for centrifuging and simultaneously conveying the cellulose material subjected to treatment towards the outlet.

A motor M is provided for the operation of the bladed rotor at peripheral speeds which vary from 15-40 meters per second.

In the inner wall of the cylindrical tubular body 1, openings 10 are provided for the insertion of the aforesaid reagent in nebulised form.

This, in particular, if an hydrogen peroxide solution employed as reagent is utilised.

A plant for producing simple sugars and/or bioethanol according to the process of the invention comprises, in addition to the turbo-reactor A, a turbo-mixer B for premixing the cellulose material or a mixture of water and cellulose material with the employed reagent, in the case the latter is, preferably, sulphuric acid or a solution of this.

In particular, the turbo-mixer B is put upstream of the turbo-reactor A and essentially comprises a cylindrical tubular body 21, closed at the opposing ends by bottoms 22, 23 and coaxially equipped with a heating/cooling jacket 24 intended to be run through by a fluid, for example diathermic oil for maintaining the inner wall of the body 21 at a predetermined temperature.

The tubular body 21 is provided with an inlet opening 25 of the cellulose material or a mixture of water and cellulose material, an inlet opening 26 of the sulphuric acid solution, and a discharge opening 27 of the cellulose material at the end of the premixing step.

For the feeding of solid flows and liquid flows, the aforesaid openings are respectively equipped with a high speed screw feeder and with controlled injection tubes, neither shown in the figures.

In the tubular body 21, a bladed rotor 28 is rotatably supported which is entirely similar to the bladed rotor 8 of the turbo-reactor A, hence it too comprises blades, here indicated with 29, and is rotatably supported in the cylindrical tubular body where it is put in rotation at peripheral speeds in the range of 15-40 m/s, so as to mix the continuous flow of cellulose material or the mixture of water and cellulose material with the sulphuric acid in a premixed flow.

The bladed rotor is driven into rotation by a motor N and it is intended to convey the cellulose material or mixture of water and cellulose material subjected to the premixing with the sulphuric acid towards the outlet 27.

At the outlet 27, the premixed cellulose material is discharged into a collection and storage hopper C which is connected to at least one feed pump D for feeding the flow of premixed cellulose material into the turbo-reactor A at a temperature in the range of about 1 to about 15 bars.

Without wishing to limit the scope of the present invention, the following example illustrates the manner in which the present invention may be carried out and used.

In particular, in the following example, specific reference is made to the bran as residue of the grinding of cereals mainly composed of seed cods, but it must be understood that other cellulose materials, such as those for example mentioned above (but not only these) can be employed in the process according to the present invention.

### COMPARATIVE EXAMPLE

To a turbo-reactor A made of special Uranus B6 alloy, whose bladed rotor is made to turn at a peripheral speed of about 35 m/s and whose inner wall is maintained at 260°C, a continuous flow of native wheat bran, with flow rate of 20 kg/hour, is fed through the opening 5.

Simultaneously, through the opening 6 and the openings 10, a 5% hydrogen peroxide solution (16.6 volumes) is continuously fed at 40 kg/hour at room temperature.

The bran has a granulometry which has the following distribution:
6% > 1400 microns
26.8% > 1000 microns
61.9% > 200 microns
3.6% > 100 microns
1.8% < 100 microns

At the inlet of the turbo-reactor A, the bran flow is mechanically dispersed into small particles which are immediately centrifuged against the inner wall of the turbo-reactor itself, where they form a dynamic, thin tubular layer.

Simultaneously, the hydrogen peroxide solution entering through the opening 6 is mechanically, finely nebulised by the blades 9 of the rotor 8, which also cause the immediate centrifugation of the smallest obtained drops. These are thus inserted in the dynamic, thin tubular layer of bran particles, with which they can interact.

The hydrogen peroxide solution introduced in nebulized form through the openings 10 further increases the interaction of the hydrogen peroxide with the bran particles.

After a residence time of about 9 minutes in the turbo-reactor A, a flow of treated bran is continuously discharged from the opening 7 with a moisture content of about 61%, with a temperature of about 100°C and with a pH of 5.6-5.7.

The treated bran presents itself at the discharge as a bleached moist mass with considerably modified fibre.

The capacity of such product to provide simple sugars was then verified by means of enzymatic hydrolysis.

The treated bran exiting from the turbo-reactor A was diluted with water 1.1 to a temperature of about 15°C, obtaining a treated bran mass at about 35°C.

A portion of said treated bran mass, in particular 200g, is further diluted with 400g of cold water, to obtain a mass which can be stirred with magnetic armature.

Then, a stirrer and the beaker containing the obtained treated bran solution are put in a stove at 40°C, where they are maintained until the end of the process for obtaining fermentable sugars.

At this point, a first quantity of enzymes is added to the aforesaid solution in the form of 2.4g of Celluclast® 1.5 L FC tq.

A subsequent first measurement of the sugars in solution carried out after about an hour from the first addition of enzymes reveals a content of sugars equal to about 26 mmol/l, i.e. 4.7 g/l.

The determination of the sugars in solution is carried out with an instrument commercialised as Accu-Check Comfort© by Roche Diagnostics, employed for personal glycemic control, which has a measurement range of 0.36-4.5 g/l.

A second and a third measurement carried out at subsequent hourly intervals do not reveal appreciable variations of the content of sugars in solution, therefore another 2.4g of Celluclast® 1.5 L FC tq are added without leading in any case to substantial variations of the sugar content calculated in the space of about an hour from the last addition.

Then 1g of Liquozyme© SC is added to the treated bran solution.

After about an hour from this addition, 32.7 mmol/l, i.e. 5.8 g/l of sugars in solution were detected.

The solution was maintained in the stove overnight, as said at 40°C, and the following morning the Accu-Check© displays overrange during repeated measurements.

The solution is therefore diluted 1.10 with water and at the subsequent measurement, the Accu-Check© shows a content of sugars of 17.5 mmol/l, i.e. 3.15 g/l.

From this last concentration, it is calculated that starting from 40g of bran (dry substance), 18.8 grams of sugars are obtained with a yield therefore of 47%.

## Claims

1. Process for the production of simple sugars starting from a cellulose material, comprising the steps of:
feeding a continuous flow of the said cellulose material or a mixture composed of said-cellulose material and water to a turbo-reactor (A) comprising a horizontal-axis cylindrical tubular body (1), provided with at least one opening (5, 6) for introducing said cellulose material or said mixture and reagents, at least one discharge opening (7) of the treated material, a heating jacket (4) for bringing the inner wall of the tubular body (1) to a predetermined temperature, a bladed rotor (8), rotatably supported in the cylindrical tubular body where it is put in rotation at speeds in the range of 15-40 m/s, so as to disperse said continuous flow of cellulose material or said mixture in a flow of cellulose material particles,
feeding into said turbo-reactor (A), in co-current with said flow of cellulose material or with said mixture, a continuous and finely subdivided flow of a reagent capable of breaking the integrity of the molecular structure of said cellulose material, making it particularly accessible for being digested during an enzymatic treatment,
centrifuging said particles of cellulose material and said reagent against the inner wall of the turbo-reactor, heated to a temperature of at least 150°C, with formation of a highly turbulent, dynamic, thin tubular fluid layer, wherein the cellulose material particles and said reagent are mechanically maintained in close contact by the blades (9) of said bladed rotor (8),
making said cellulose material and said reagent react in said thin layer while it advances in substantial contact with said inner wall of the turbo-reactor (A) towards the discharge opening (7),
continuously discharging a flow of treated cellulose material,
sending said flow of treated cellulose material to an enzymatic digestion step with the addition of a sufficient quantity of enzymes, said enzymes being specific for hydrolysing said treated cellulose material with the obtainment of said simple sugars,
wherein said flow of cellulose material or said mixture and said reagent flow are premixed with each other in a turbo-mixer (B) placed upstream of said turbo-reactor (A), said turbo-mixer (B) comprising a horizontal-axis cylindrical tubular body (21), provided with inlet openings (25, 26) of said cellulose material or said mixture and said reagent, at least one discharge opening (27) of the cellulose material premixed with said reagent, a heating/cooling jacket (24) for bringing the inner wall of the tubular body (21) to a predetermined temperature in the range of 10-100°C, and a bladed rotor (28), rotatably supported in the cylindrical body where it is put in rotation at peripheral speeds in the range of 15-40 m/s, so as to premix said continuous flow of cellulose material or said mixture with said continuous reagent flow in a continuous flow of premixed material.

2. Process according to claim 1, wherein said continuous flow of premixed material discharged from said turbo-mixer (B) is stored in a hopper (C) connected to at least one feed pump (D) for feeding said continuous flow of premixed cellulose material into said turbo-reactor (A) at a pressure in the range of about 1 to about 15 bars.

3. Process according to any one of the preceding claims, wherein said reagent capable of breaking the integrity of the molecular structure of said cellulose material is sulphuric acid or a solution of sulphuric acid.

4. Process according to any one of the preceding claims, wherein said sulphuric acid is fed in a percentage in the range of 1-5% in relation to the weight of the cellulose material.

5. Process according to any one of the preceding claims, comprising, before said enzymatic digestion step, a neutralisation step in which said treated cellulose material which has been discharged by said turbo-reactor (A) is made to react with an oxide or alkaline hydroxide or earth alkaline.

6. Process according to claim 1, wherein said reagent capable of breaking the integrity of the molecular structure of said cellulose material is an hydrogen peroxide solution.

7. Process according to claim 6, wherein said hydrogen peroxide solution is fed at a pressure in the range of about 1 to about 15 bars.

8. Process according to claim 6 or 7, wherein said hydrogen peroxide solution consists of hydrogen peroxide of at least 10 volumes (3% titre).

9. Process according to claim 8, wherein said hydrogen peroxide solution consists of hydrogen peroxide of 30-40 volumes.

10. Process according to any one of the claims 6-9, wherein the weight ratio between hydrogen peroxide and cellulose material varies from 1:2 to 1:20.

11. Process according to any one of the preceding claims, wherein the residence time of said flow of cellulose material or said mixture of water and cellulose material with said reagent flow in said turbo-reactor (A) is a time which varies in the range of about 2 to about 30 minutes.

12. Process according to any one of the preceding claims, wherein said turbo-reactor (A) is a substantially sealed reactor capable of maintaining an internal pressure up to a value of about 15 bars.

13. Process according to any one of the preceding claims, wherein said enzymatic digestion step is carried out at a temperature in the range of 35-45°C, preferably at 40°C.

14. Process according to any one of the preceding claims, wherein said enzymatic digestion step is carried out for a time which varies in the range of 10 minutes to 36 hours.

15. Process according to any one of the preceding claims, wherein the inner wall of said turbo-reactor is heated to a temperature in the range of about 230 to about 300°C, preferably about 260°C.

16. Process according to any one of the preceding claims, wherein said enzymatic digestion step is followed by or carried out simultaneously with a step of alcoholic fermentation, with consequent transformation of said simple sugars into ethanol.

17. Use of a plant for the production of simple sugars starting from a cellulose material with the process of any one of the preceding claims, said plant comprising:
a turbo-reactor (A) comprising a horizontal-axis cylindrical tubular body (1), provided with at least one opening (5, 6) for introducing said cellulose material or said mixture and reagents, at least one discharge opening (7) of the treated product, a heating jacket (4) for bringing the inner wall of the tubular body (1) to a predetermined temperature, a bladed rotor (8) rotatably supported in the cylindrical tubular body, said turbo-reactor (A) being a substantially sealed reactor capable of maintaining an internal pressure up to a value of about 15 bars,
and a turbo-mixer (B) connected upstream to said turbo-reactor (A) and comprising a horizontal-axis cylindrical tubular body (21), provided with inlet openings (25, 26), at least one discharge opening (27), a cooling/heating jacket (24) for bringing the inner wall of the tubular body (21) to a predetermined temperature, and a bladed rotor (28), rotatably supported in the cylindrical tubular body.

18. Use according to claim 17, in which said plant further comprises a hopper (C) placed downstream of said turbo-mixer (B) and a pump (D) connected to said hopper (C) and placed upstream of said turbo-reactor (A) respectively for the collection of said flow of premixed cellulose material discharged from said turbomixer (B) and for feeding said collected flow into said turbo-reactor (A) at a pressure in the range of about 1 to about 15 bars.

## Patentansprüche

1. Verfahren zur Herstellung von einfachen Zuckern aus Zellulose-Ausgangsmaterial, umfassend die Schritte:
Zuführen eines kontinuierlichen Stroms des Zellulosematerials oder eines Gemischs bestehend aus dem Zellulosematerial und Wasser zu einem Turboreaktor (A), umfassend einen zylindrischen rohrförmigen Körper (1) mit horizontaler Achse mit mindestens einer Eintrittsöffnung (5, 6) zum Einbringen des Zellulosematerials oder des Gemisches und von Reagenzien, mindestens einer Austrittsöffnung (7) für das behandelte Material, einen Heizmantel (4), um die Innenwand des rohrförmigen Körpers (1) auf eine vorbestimmte Temperatur zu bringen, einen mit Schaufeln versehenen Rotor (8), der drehbar in dem zylindrischen Rohrkörper gelagert ist, wo er mit einer Geschwindigkeit im Bereich von 15-40 m/s in Rotation gesetzt wird, um so den kontinuierlichen Strom von Zellulosematerial oder die Mischung zu einem Strom von Zellulosematerialpartikeln zu dispergieren,
Einspeisen in den Turboreaktor (A) im Gegenstrom zu dem Strom von Zellulosematerial oder der Mischung eines kontinuierlichen und fein verteilten Stroms eines Reagens, das in der Lage ist, die molekulare Struktur des Zellulosematerials aufzubrechen, so dass es besonders zugänglich für ein Aufschließen während einer enzymatischen Behandlung ist,
Zentrifugieren der Teilchen aus Zellulosematerial und des Reagens gegen die Innenwand des Turboreaktors, die auf eine Temperatur von mindestens 150°C aufgeheizt ist, unter Bildung einer stark turbulenten, dynamischen dünnen tubulären Fluidschicht, wobei die Zellulosematerialpartikel und das Reagens durch die Schaufeln (9) des mit Schaufeln versehenen Rotors mechanisch in einem engen Kontakt gehalten werden (8),
Reagierenlassen des Zellulosematerials und des Reagens in der dünnen Schicht, während sie sich in erheblichem Kontakt mit der Innenwand des Turboreaktors (A) in Richtung der Austrittsöffnung (7) vorbewegt,
kontinuierliches Abziehen eines Stroms von behandelten Zellulosematerial,
wobei der Strom des behandelten Zellulosematerials einem enzymatischen Verdauungsschritt unter Zugabe einer ausreichenden Menge an Enzymen zugeleitet wird, wobei die Enzyme spezifisch für das Hydrolysieren des Zellulosematerials unter Erhalt der einfachen Zucker ist,
wobei der Strom von Zellulosematerial oder des Gemischs und des Reagens in einem Turbomischer (B) miteinander vorgemischt werden, der stromaufwärts des Turboreaktors (A) angeordnet ist, wobei der Turbomischer (B) einen zylindrischen rohrförmigen Körper (21) mit horizontaler Achse umfasst mit Eintrittsöffnungen (25, 26) zum Einbringen des Zellulosematerials oder des Gemisches und von Reagenzien, mindestens einer Austrittsöffnung (27) für das mit dem Reagens vorgemischte Zellulosematerial, einem Heizmantel (24), um die Innenwand des rohrförmigen Körpers (21) auf eine vorbestimmte Temperatur im Bereich von 10-100°C zu bringen, einem mit Schaufeln versehenen Rotor (28), der drehbar in dem zylindrischen Rohrkörper gelagert ist, wo er mit einer Geschwindigkeit im Bereich von 15-40 m/s in Rotation gesetzt wird, um so den kontinuierlichen Strom von Zellulosematerial oder der Mischung mit dem kontinuierlichen Strom von Reagens zu einem kontinuierlichen Strom von vorgemischtem Material vorzumischen.

2. Verfahren nach Anspruch 1, wobei der kontinuierliche Strom von vorgemischtem Material, der aus dem Turbomischer (B) entnommen wird, in einem Bunker (C) gespeichert wird, der mit mindestens einer Förderpumpe (D) zum Zuführen des kontinuierlichen Stroms von vorgemischten Zellulosematerial unter einem Druck im Bereich von etwa 1 bis etwa 15 bar in den Turboreaktor (A) verbunden ist.

3. Verfahren nach einem der vorhergehenden Ansprüche, wobei das Reagens zum Aufbrechen der molekularen Struktur des Zellulosematerials Schwefelsäure oder eine Schwefelsäurelösung ist.

4. Verfahren nach einem der vorhergehenden Ansprüche, wobei die Schwefelsäure in einem Anteil im Bereich von 1-5% bezogen auf das Gewicht des Zellulosematerials zugeführt wird.

5. Verfahren nach einem der vorhergehenden Ansprüche, umfassend vor dem Schritt der enzymatischem Verdauung einen Neutralisierungsschritt, während dessen das behandelte Zellulosematerial, das aus dem Turboreaktor (A) entnommen wird, mit einem Oxid oder alkalischen oder erdalkalischen Hydroxid reagiert.

6. Verfahren nach Anspruch 1, wobei das Reagens zum Aufbrechen der molekularen Struktur des Zellulosematerials eine Wasserstoffperoxidlösung ist.

7. Verfahren nach Anspruch 6, wobei die Wasserstoffperoxidlösung unter einem Druck im Bereich von etwa 1 bis etwa 15 bar zugeführt wird.

8. Verfahren nach Anspruch 6 oder 7, wobei die Wasserstoffperoxidlösung aus mindestens 10 Volumen Wasserstoffperoxid besteht (3% Titer).

9. Verfahren nach Anspruch 8, wobei die Wasserstoffperoxidlösung aus 30-40 Volumen Wasserstoffperoxid besteht.

10. Verfahren nach einem der Ansprüche 6-9, wobei das Gewichtsverhältnis zwischen Wasserstoffperoxid und Zellulosematerial von 1:2 bis 1:20 variiert.

11. Verfahren nach einem der vorhergehenden Ansprüche, wobei die Verweilzeit der Strömung des Zellulosematerials oder des Gemisches aus Wasser und Zellulosematerial, das mit dem Strom des Reagens in dem Turboreaktor (A) eine Zeit ist, die in dem Bereich von 2 bis etwa 30 Minuten variiert.

12. Verfahren nach einem der vorhergehenden Ansprüche, wobei der Turboreaktor (A) ein im Wesentlichen abgedichteter Reaktor ist, der in der Lage ist, einen Innendruck bis zu einem Wert von etwa 15 bar aufrechtzuerhalten.

13. Verfahren nach einem der vorhergehenden Ansprüche, bei dem der enzymatische Verdauungsschritt bei einer Temperatur im Bereich von 35-45°C durchgeführt wird, vorzugsweise bei 40°C.

14. Verfahren nach einem der vorhergehenden Ansprüche, wobei der enzymatische Verdauungsschritt während einer Zeit durchgeführt wird, die von 10 Minuten bis zu 36 Stunden variiert.

15. Verfahren nach einem der vorhergehenden Ansprüche, wobei die Innenwand des Turboreaktors auf eine Temperatur im Bereich von etwa 230 bis etwa 300°C erhitzt wird, vorzugsweise auf etwa 260°C.

16. Verfahren nach einem der vorhergehenden Ansprüche, bei dem nach dem oder während des enzymatischen Verdauungsschritts ein Schritt der alkoholischen Gärung durchgeführt wird, wobei die einfachen Zucker zu Ethanol umgesetzt werden.

17. Verwendung einer Anlage zur Herstellung von einfachen Zuckern aus Zellulosematerial, umfassend:
einen Turboreaktor (A), umfassend einen zylindrischen rohrförmigen Körper (1) mit horizontaler Achse mit mindestens einer Eintrittsöffnung (5, 6) zum Einbringen des Zellulosematerials oder des Gemisches und von Reagenzien, mindestens einer Austrittsöffnung (7) für das behandelte Material, einen Heizmantel (4), um die Innenwand des rohrförmigen Körpers (1) auf eine vorbestimmte Temperatur zu bringen, einen mit Schaufeln versehenen Rotor (8), der drehbar in dem zylindrischen Rohrkörper gelagert ist, wobei der Turboreaktor (A) ein im Wesentlichen abgedichteter Reaktor ist, der in der Lage ist, einen Innendruck bis zu einem Wert von etwa 15 bar aufrechtzuerhalten.
und einen Turbomischer (B), der stromaufwärts des Turboreaktors (A) angeordnet ist, umfassend einen zylindrischen rohrförmigen Körper (21) mit horizontaler Achse mit Eintrittsöffnungen (25, 26), mindestens einer Austrittsöffnung (27), einem Kühl-/Heizmantel (24), um die Innenwand des rohrförmigen Körpers (21) auf eine vorbestimmte Temperatur zu bringen, und einen mit Schaufeln versehenen Rotor (28), der drehbar in dem zylindrischen Rohrkörper gelagert ist.

18. Verwendung nach Anspruch 18, wobei die Anlage ferner einen Bunker (C), der stromabwärts von dem Turbomischer (B) angeordnet ist, und eine Pumpe (D) umfasst, die mit dem Bunker (C) verbunden ist und stromaufwärts des Turboreaktors (A) angeordnet ist, die zum Sammeln des Stroms von vorgemischten Zellulosematerial, welcher aus dem Turbomischer (B) entnommen wird, beziehungsweise zum Zuführen dieses gesammelten Stroms in den Turboreaktor (A) bei einem Druck im Bereich von etwa 1 bis etwa 15 bar dienen.

## Revendications

1. Procédé pour la production de sucres simples à partir d'un matériau cellulosique, comprenant les étapes consistant :
à introduire un flux continu du matériau cellulosique, ou d'un mélange composé du matériau cellulosique et d'eau, dans un turboréacteur (A) comprenant un corps tubulaire (1) cylindrique d'axe horizontal et pourvu d'au moins une ouverture (5, 6) pour introduire le matériau cellulosique, ou le mélange, et des réactifs, au moins une ouverture d'évacuation (7) du matériau traité, une enveloppe chauffante (4) pour amener la paroi interne du corps tubulaire (1) à une température prédéterminée, un rotor à pales (8) supporté de manière rotative dans le corps tubulaire cylindrique où il est mis en rotation à une vitesse située dans la fourchette comprise entre 15 et 40 m/s de façon à disperser le flux continu de matériau cellulosique, ou de mélange, en un flux de particules de matériau cellulosique,
à introduire dans le turboréacteur (A), à contre-courant du flux de matériau cellulosique, ou de mélange, un flux continu et finement divisé d'un réactif capable de rompre l'intégrité de la structure moléculaire du matériau cellulosique en le rendant particulièrement apte à être digéré au cours d'un traitement enzymatique,
à centrifuger les particules de matériau cellulosique et le réactif contre la paroi interne du turboréacteur, chauffée à une température d'au moins 150 °C, avec formation d'une fine couche de fluide tubulaire à forte turbulence et dynamique, les particules de matériau cellulosique et le réactif étant maintenus mécaniquement en contact intime par les pales (9) du rotor à pales (8),
à amener le matériau cellulosique et le réactif à réagir dans la couche mince pendant qu'elle avance en direction de l'ouverture d'évacuation (7) en étant en contact substantiel avec la paroi interne du turboréacteur (A),
à décharger en continu un flux de matériau cellulosique traité,
à envoyer le flux de matériau cellulosique traité vers une étape de digestion enzymatique avec addition d'une quantité suffisante d'enzymes, les enzymes étant spécifiques à l'hydrolyse du matériau cellulosique traité pour l'obtention des sucres simples,
dans lequel procédé le flux de matériau cellulosique, ou de mélange, et le flux de réactif sont pré-mélangés les uns aux autres dans un turbomélangeur (B) placé en amont du turboréacteur (A), le turbomélangeur (B) comprenant un corps tubulaire (21) cylindrique d'axe horizontal et étant pourvu d'ouvertures d'introduction (25, 26) du matériau cellulosique, ou du mélange, et du réactif, au moins une ouverture d'évacuation (27) du matériau cellulosique pré-mélangé avec le réactif, une enveloppe chauffante/-refroidissante (24) pour amener la paroi interne du corps tubulaire (21) à une température prédéterminée située dans la plage comprise entre 10 et 100 °C, et un rotor à pales (28) supporté de manière rotative dans le corps cylindrique où il est mis en rotation à des vitesses périphériques situées dans la plage comprise entre 15 et 40 m/s de façon à pré-mélanger le flux continu de matériau cellulosique, ou le mélange, avec le flux continu de réactif en un flux continu de matériau pré-mélangé.

2. Procédé selon la revendication 1, dans lequel le flux continu de matériau pré-mélangé déchargé du turbomélangeur (B) est stocké dans une trémie (C) connectée à au moins une pompe d'alimentation (D) pour introduire le flux continu du matériau cellulosique pré-mélangé dans le turboréacteur (A) à une pression située dans la fourchette comprise entre environ 1 à environ 15 bars.

3. Procédé selon l'une quelconque des revendications précédentes, dans lequel le réactif capable de rompre l'intégrité de la structure moléculaire du matériau cellulosique est de l'acide sulfurique ou une solution d'acide sulfurique.

4. Procédé selon l'une quelconque des revendications précédentes, dans lequel l'acide sulfurique est introduit dans une proportion située dans la fourchette comprise entre 1 à 5 % par rapport au poids du matériau cellulosique.

5. Procédé selon l'une quelconque des revendications précédentes, comprenant, avant l'étape de digestion enzymatique, une étape de neutralisation dans laquelle le matériau cellulosique traité qui a été déchargé du turboréacteur (A) est amené à réagir avec un oxyde ou un hydroxyde alcalin ou un alcalino-terreux.

6. Procédé selon la revendication 1, dans lequel le réactif capable de rompre l'intégrité de la structure moléculaire du matériau cellulosique est une solution de peroxyde d'hydrogène.

7. Procédé selon la revendication 6, dans lequel la solution de peroxyde d'hydrogène est introduite à une pression située dans la fourchette comprise entre environ 1 et environ 15 bars.

8. Procédé selon la revendication 6 ou 7, dans lequel la solution de peroxyde d'hydrogène consiste en du peroxyde d'hydrogène d'au moins 10 volumes (titre 3 %).

9. Procédé selon la revendication 8, dans lequel la solution de peroxyde d'hydrogène consiste en du peroxyde d'hydrogène de 30-40 volumes.

10. Procédé selon l'une quelconque des revendications 6 à 9, dans lequel le rapport pondéral entre le peroxyde d'hydrogène et un matériau cellulosique varie de 1:2 et 1:20.

11. Procédé selon l'une quelconque des revendications précédentes, dans lequel le temps de séjour du flux de matériau cellulosique, ou du mélange d'eau et de matériau cellulosique, avec le flux de réactif dans le turboréacteur (A) est un temps qui varie dans la fourchette comprise entre environ 2 et environ 30 minutes.

12. Procédé selon l'une quelconque des revendications précédentes, dans lequel le turboréacteur (A) est un réacteur sensiblement étanche apte à maintenir une pression interne allant jusqu'à une valeur d'environ 15 bars.

13. Procédé selon l'une quelconque des revendications précédentes, dans lequel l'étape de digestion enzymatique est effectuée à une température située dans la fourchette comprise entre 35 et 45 °C, de préférence à 40 °C.

14. Procédé selon l'une quelconque des revendications précédentes, dans lequel l'étape de digestion enzymatique est effectuée pendant une durée qui varie dans la fourchette comprise entre 10 minutes et 36 heures.

15. Procédé selon l'une quelconque des revendications précédentes, dans lequel la paroi interne du turboréacteur est chauffée à une température située dans la fourchette comprise entre environ 230 et environ 300 °C, de préférence à environ 260 °C.

16. Procédé selon l'une quelconque des revendications précédentes, dans lequel l'étape de digestion enzymatique est suivie ou réalisée simultanément à une étape de fermentation alcoolique, avec une transformation consécutive des sucres simples en éthanol.

17. Utilisation d'une installation pour la production de sucres simples à partir d'un matériau cellulosique avec le procédé selon l'une quelconque des revendications précédentes, ladite installation comprenant :
un turboréacteur (A) comprenant un corps tubulaire (1) cylindrique d'axe horizontal et pourvu d'au moins une ouverture (5, 6) pour introduire le matériau cellulosique, ou le mélange, et des réactifs, au moins une ouverture d'évacuation (7) du matériau traité, une enveloppe chauffante (4) pour amener la paroi interne du corps tubulaire (1) à une température prédéterminée, un rotor à pales (8) supporté de manière rotative dans le corps tubulaire cylindrique, le turboréacteur (A) étant un réacteur sensiblement étanche apte à maintenir une pression interne allant jusqu'à une valeur d'environ 15 bars,
et un turbomélangeur (B) connecté en amont du turboréacteur (A) et comprenant un corps tubulaire (21) cylindrique d'axe horizontal et pourvu d'ouvertures d'introduction (25, 26), au moins une ouverture d'évacuation (27), une enveloppe refroidissante/chauffante (24) pour amener la paroi interne du corps tubulaire (21) à une température prédéterminée, et un rotor à pales (28) supporté de manière rotative dans le corps tubulaire cylindrique.

18. Utilisation selon la revendication 17, dans laquelle l'installation comprend en outre une trémie (C) placée en aval du turbomélangeur (B) et une pompe (D) connectée à la trémie (C) et placée en amont du turboréacteur (A), respectivement, pour la collecte du flux de matériau cellulosique pré-mélangée déchargée du turbomélangeur (B) et pour introduire le flux collecté dans le turboréacteur (A) à une pression située dans la fourchette comprise entre environ 1 et environ 15 bars.
